# EUROPÄISCHE PATENTSCHRIFT

(11) **EP 0 837 674 B1**
(45) Veröffentlichungstag und Bekanntmachung des Hinweises auf die Patenterteilung: **19.12.2001**
(21) Anmeldenummer: 96922890.7
(22) Anmeldetag: 24.06.1996
(51) Int. Cl.: A61K 9/24

(54) **VERFAHREN ZUR HERSTELLUNG VON SCHICHTTABLETTEN ZUR KONTROLLIERTEN FREISETZUNG VON WIRKSTOFFEN**
PROCESS FOR PRODUCING A LAYERED TABLET FOR THE CONTROLLED RELEASE OF ACTIVE SUBSTANCES
PROCEDE POUR LA PREPARATION D'UN COMPRIME STRATIFIE POUR LA LIBERATION CONTROLEE DE SUBSTANCES ACTIVES

(30) Priorität: 07.07.1995 DE 19524753
(43) Veröffentlichungstag der Anmeldung: 29.04.1998
(73) Patentinhaber: LTS Lohmann Therapie-Systeme AG, 56626 Andernach (DE)
(72) Erfinder: CREMER, Karsten, D-53119 Bonn (DE)
(74) Vertreter: Flaccus, Rolf-Dieter, Dr.
(86) Internationale Anmeldenummer: EP9602736
(87) Internationale Veröffentlichungsnummer: WO9702812

(56) Entgegenhaltungen:
- DE-A- 4 341 442
- DE-A- 4 416 926
- VOIGT ET AL.: 'Lehrbuch der pharmazeutischen Technologie', Seiten 223-225, 5. Ausg., 1984, VERLAG CHEMIE, WEINHEIM

## Beschreibung

Verfahren zur Herstellung einer Schichttablette zur kontrollierten Freisetzung von Wirkstoffen in einem flüssigen Medium mit mindestens einer wirkstoffhaltigen, Kontaktflächen gegenüber dem flüssigen Medium aufweisenden schichtförmigen Matrix, deren Kontaktflächen zum Teil eine die Wirkstofffreisetzung verzögernde oder verhindernde Abdeckschicht aufweisen, wobei die wirkstoffhaltige Matrix und die erodierbare Abdeckschicht Dickegradienten besitzen, deren Richtungen komplementär zueinander verlaufen, dadurch gekennzeichnet, daß
a) mittels eines speziell geformten Stempelwerkzeugs die Matrix oder die erodierbare Abdeckschicht mit einem Dickegradienten als erster Teilkörper erzeugt wird, der

a) mit vakuumbeaufschlagten Transferelementen in die Matrizenöffnung einer Tablettiermaschine transferiert wird, und
b) das Pulver- oder Granulat-Material für die den ersten Teilkörper ergänzende(n) Abdeckschicht(en) oder Matrix zum ersten Teilkörper gegeben und zusammengepresst wird.

Die Erfindung betrifft insbesondere Verfahren zur Herstellung von Schichttabletten zur kontrollierten Freisetzung von pharmazeutischen Wirkstoffen in den Säften des Gastrointestinaltraktes oder in Flüssigkeiten, die zur Prüfung von Arzneimitteln geeignet sind.

Sie umfaßt schließlich die Verwendung solcher Verfahren zur Herstellung einer bezüglich Herstellung und Gebrauch besonders günstigen Tablettenform.

Als Schichttabletten werden beim Stand der Technik in der Pharmazeutischen Technologie Tabletten bezeichnet, die aus verschiedenen, fest aneinander haftenden, parallelen bzw. konzentrisch gewölbten Schichten aus komprimierten Pulver-oder Granulatpartikeln bestehen (vgl. Hunnius Pharmazeutisches Wörterbuch, 6. Auflage, Berlin 1986). Im Gegensatz zu Manteltabletten, welche als Tabletten mit einem vorgepressten Kern und einem ihn lückenlos umgebenden, ebenfalls durch Pressen von Pulver oder Granulat erzeugten Material definiert sind, werden konventionelle Schichttabletten in einem Arbeitsgang und auf einer Tablettenpresse durch mehrmals aufeinanderfolgendes Pressen unterschiedlicher Teilchenarten gefertigt, wobei - bedingt durch die Verwendung desselben Presswerkzeugs bei jeder Pressung - die Schichten eine charakteristische Form erhalten. Die Formen der einzelnen Schichten können also nicht unabhängig voneinander frei gewählt werden. Die Verwendung von biplanen Stempelwerkzeugen führt zu Schichttabletten mit durchweg parallelen Schichten, wenn man von den Randbezirken im Bereich der optionalen Facette absieht. Bei der Verwendung von gewölbten Stempeln wird die untere, zuerst gepresste Schicht bikonvex, während die obere bzw. zweite Schicht eine gleichmäßige Dicke besitzt und parallel zur gewölbten oberen Begrenzungsfläche der zuerst gepressten Schicht verläuft.

Schichttabletten und ein Verfahren zu ihrer Herstellung wurden erstmals 1917 beschrieben (US-Patent 1,248,571). Sie werden in der Pharmazie aus verschiedenen Überlegungen heraus eingesetzt. Zum einen bieten sie die Möglichkeit, inkompatible Wirkstoffe räumlich voneinander getrennt in einer Tablette zu kombinieren. Zum anderen erlauben sie die Kombination mehrerer Rezepturen mit unterschiedlichen Freisetzungseigenschaften. So läßt sich zum Beispiel die eine Schicht zur Abgabe einer Initialdosis des Wirkstoffes als schnellzerfallende, schnellfreisetzende Formulierung einsetzen, während die zweite Schicht eine Erhaltungsdosis in retardierter Form enthält.

In jüngerer Zeit wurden spezielle Schichttabletten zur kontrollierten Freisetzung von Wirkstoffen, insbesondere zur Freisetzung nach nullter Ordnung beschrieben (U. Conte et al., J. Controlled Rel. 26, S. 39-47, 1993). Diese Schichttabletten enthalten eine wirkstoffhaltige Quellmatrix und mindestens eine auf diese aufgepresste Hilfsstoffschicht, welche die Oberfläche der Matrix auf einer Seite bedeckt. Die Hilfsstoffschicht ist weitgehend inert und wirkstoffundurchlässig. Die wirkstoffhaltige Matrix setzt den Wirkstoff in flüssigen Medien durch Diffusion frei.

Die Quellung der Matrix im wässrigen Medium soll sicherstellen, daß sich die Freisetzungsgeschwindigkeit im Verlauf der Wirkstoffreisetzung - bedingt durch die progrediente Verarmung der Matrix an Wirkstoff und die gleichzeitige Verlängerung des Diffusionsweges - nicht verringert, sondern im Sinne eines Prozesses nullter Ordnung aufrechterhalten bleibt. Diese Quellung bewirkt eine wesentliche Vergrößerung der Matrixoberfläche gegenüber ihrem Trockenzustand. Die bisher veröffentlichten Daten über das tatsächliche Freisetzungsverhalten dieser Schichttabletten zeigen jedoch, daß in der Praxis der Quellungseffekt zum Aufrechterhalten der anfänglichen Freisetzungsgeschwindigkeit meist nicht ausreicht. Wird andererseits die Quellmatrix derart formuliert, daß sie in einem wässrigen Medium sehr stark quillt, führt dies fast unweigerlich zu einer mangelnden Kohäsion der Tablette: Sie zerfällt und bewirkt dabei ein "dose dumping", d.h. eine plötzliche Freisetzung des restlichen Wirkstoffes.

Bei anderen Darreichungsformen als Schichttabletten wurde das Gestaltungsprinzip der kontinuierlichen Vergrößerung der Kontaktfläche einer Matrix zum flüssigen Freisetzungsmedium zur Kontrolle der Freisetzungsgeschwindigkeit auf eine andere Weise umgesetzt. Für Vorrichtungen aus der Gattung der umhüllten festen Arzneiformen (coated tablets and capsules), wie sie aus der üblichen Dragier- oder Filmcoatingverfahren hervorgehen, beschreibt die Anmeldeschrift WO 94/0747 zumindest ansatzweise die Verwendung einer Umhüllung, welche eine an verschiedenen Stellen unterschiedliche Dicke aufweist und aus einem in flüssigem Medium erodierbaren Material besteht. Im Verlaufe der Wirkstoffreisetzung erodiert die Umhüllung, ein Vorgang, der an den dünnsten Stellen der Umhüllung zuerst abgeschlossen ist und kontinuierlich oder diskontinuierlich voranschreitet: Die Flächenausdehnung der Umhüllung wird geringer, im Gegenzug nimmt die Kontaktfläche des Kerns zum Freisetzungsmedium zu.

Die WO 94/0747 beschreibt mit einem Überzug ummantelte Tabletten. Sie zeigt jedoch keine Möglichkeit auf, wie die zur Nutzung des Effektes der Grenzflächenvergrößerung notwendigen Dickgradienten der Ummantelung gezielt herzustellen sind. Die konventionellen Umhüllungsverfahren, auf die als Herstellungsmethode verwiesen wird, führen bei bestimmten Kernformen zwangsweise zu leicht ungleichmäßigen Dicken. So kann z.B. nicht verhindert werden, daß beim Umhüllen von Tabletten oder Kapseln die Filmdicke an den Kanten und an Orten mit kleinem Wölbungsradius unterdurchschnittlich gering ist. In den Beispielen 1 und 4 wurden die konventionellen Umhüllungsverfahren nicht einmal variiert, um ungleichmäßige Dicken zu erzielen. Es ist auch nicht zu erwarten, daß solche Umhüllungsverfahren (verwiesen wird auf Wirbelschicht- und Dragierkesselverfahren) derart gestaltet werden können, daß die fur eine kontinuierliche Oberflächenvergrößerung des Kerns notwendigen Dickegradienten der erodierbaren Umhüllung gezielt erzeugt werden können.

Angesichts des Mangels an geeigneten Herstellungsverfahren für solche Arzneiformen mit erodierbaren Umhüllungen mit Dickegradienten besteht ein Bedarf an alternativen Lösungen, welche nicht mehr auf dem Gestaltungsprinzip mit einem Kern und einer Umhüllung beruhen.

Die deutsche Anmeldeschrift P 43 41 442.7 vermittelt - wenn auch vorwiegend durch die gewählten Abbildungen - bereits die Lehre, daß sich Dickegradienten zur Erzielung des oben beschriebenen Effektes auch durch den Aufbau der Arzneiform in ungefähr parallelen Schichten realisieren lassen, wobei sie allerdings keine Hinweise auf eine bevorzugte Ausgestaltung, z.B. in Form eines Koextrudats oder mehrerer miteinander verklebter Presslinge gibt. Sie enthält im Falle der Verwendung von Presslingen für die wirkstoffhaltige Matrix oder für die erodierbare Hilfsstoffschicht - außer dem Hinweis, daß ein adhäsionsvermittelndes Hilfsmaterial verwendet werden sollte, falls die Schichten nicht von selbst aufeinander haften - auch keinen produktionstechnisch verwertbaren Hinweis darauf, welcher Art ein geeignetes Herstellungsverfahren bzw. die Ausgestaltung der Vorrichtung sein sollte, damit die Presslinge wie etwa im Fall einer Schichttablette effizient zu einer Arzneiform kombiniert bzw. zusammengefügt werden können.

Aufgabe der vorliegenden Erfindung ist daher die Herstellung einer Schichttablette zur kontrollierten Freisetzung von Wirkstoffen aus einer schichtförmigen, wirkstoffhaltigen Matrix, die problemlos mit bekannten Tablettenpressen herstellbar ist, und deren Abdeckschicht die Wirkstofffreisetzungsgeschwindigkeit in einem flüssigen Medium kontrolliert, jedoch ohne die oben erwähnten Nachteile des Standes der Technik zu besitzen.

Gelöst wird die Aufgabe bei einem Verfahren entsprechend dem Oberbegriff von Anspruch 1 mit der Erfindung durch eine Ausbildung des Verfahrens entsprechend dem Kennzeichnungsteil von Anspruch 1.

Eine erfindungsgemäße Schichttablette gehört zu jener Gruppe von Schichttabletten, welche eine wirkstoffhaltige, schichtförmige Matrix und eine auf dieser Matrix aufliegende, die Wirkstofffreisetzung kontrollierende Abdeckschicht besitzen. Im Gegensatz zu den aus dem Stand der Technik bekannten Schichttabletten dieser Gruppe handelt es sich bei der Abdeckschicht jedoch um wenigstens eine mit Dickegradienten aufliegende, durch Aufpressen von pulver- oder granulatförmigem, in flüssigen Medien erodierbaren Material aufgebrachte Schicht.

Erfindungsgemäß wird das Prinzip der Steuerung der Freisetzungsgeschwindigkeit durch eine mittels Dickegradienten kontrollierte Erosion des Materials einer Abdeckschicht auf die Gattung der Schichttabletten angewendet. Die Freisetzungsgeschwindigkeit von Wirkstoff wird bei einer solchen Schichttablette durch ihre geometrische Form, insbesondere durch die Dickegradienten der Abdeckschicht sowie durch deren Erosionsgeschwindigkeit gesteuert.

Zu Beginn der Freisetzung diffundiert der Wirkstoff aus der Randzone der wirkstoffhaltigen Matrixschicht in das diese umgebende Freisetzungsmedium. Die Abdeckschicht stellt hingegegen zunächst eine Diffusionsbarriere dar.

Im Verlauf der Freisetzung verarmen die Randzonen der wirkstoffhaltigen Matrixschicht an Wirkstoff. Verlängerte Diffusionswege zur Kontaktfläche mit dem Freisetzungsmedium und verringerte Konzentrationsgradienten an der Kontaktfläche wirken sich negativ auf die Freisetzungsgeschwindigkeit aus. Gleichzeitig kommt es jedoch zu einem kompensatorischen Effekt. Die Abdeckschicht erodiert, dadurch vergrößert sich die für die Freisetzung verfügbare Kontaktfläche zum Freisetzungsmedium. Bei geeigneter Wahl der Dickegradienten können also diese zwei einander entgegengesetzten Einflüsse auf die Freisetzungsgeschwindigkeit zum Ausgleich gebracht werden, so daß eine Kinetik nullter Ordnung resultiert, die in der Therapie häufig wünschenswert ist.

Die Dickegradienten können jedoch auch anders gewählt werden, etwa so, daß erst gegen Ende des Freisetzungsverlaufes eine wesentliche Vergrößerung der Kontaktfläche zwischen wirkstoffhaltiger Matrix und Freisetzungsmedium erfolgt.

Vorteilhafterweise lässt sich eine erfindungsgemäße Schichttablette auch so ausgestalten, daß zu beiden Seiten der wirkstoffhaltigen Matrixschicht Pulver oder Granulat zur Erzeugung einer die Wirkstofffreisetzung steuernde Abdeckschicht aufgepresst wird. Hierdurch resultiert eine Dreischichttablette, welche sich im äußeren Erscheinungsbild von herkömmlichen Dreischichttabletten insbesondere durch die gezielt eingebrachten Dickegradienten der Schichten unterscheidet. Dabei ist der Steuerungseinfluss der Abdeckschichten auf die Freisetzungsgeschwindigkeit noch größer als bei nur einer Abdeckschicht, da der potentielle Zuwachs an Kontakfläche zwischen Matrixschicht und flüssigem Medium größer ist.

Sollen in der Schichttablette zwei oder mehr Wirkstoffe enthalten sein und in kontrollierter Weise freigesetzt werden, kann es vorteilhaft oder notwendig werden, auch die wirkstoffhaltige Matrix mit mehr als einer Schicht auszubilden. Dadurch ist es möglich, unterschiedlich schnell durch die Matrixschichten diffundierende Wirkstoffe dennoch mit vorgegebenen Geschwindigkeiten freizusetzen. Dabei können unterschiedliche Freisetzungsgeschwindigkeiten - ob für einen oder mehrere Wirkstoffe - verwirklicht werden. So kann etwa eine Wirkstoffmenge relativ schnell im Sinne einer Initialdosis und eine weitere Wirkstoffmenge retardiert im Sinne einer Erhaltungsdosis aus unterschiedlichen wirkstoffhaitigen Matrixschichten freigesetzt werden.

Die Geometrie der wirkstoffhaltigen Matrixschicht und die der Abdeckschicht werden so aufeinander abgestimmt, daß beide Schichten Dickegradienten besitzen, deren Richtungen komplementär zueinander verlaufen. Durch eine solche Konstruktion kann die Schichttablette insgesamt eine konventionelle biplane oder biokonvexe Form annehmen. Dadurch bleibt die Schichttablette zum einen auf allen gängigen Maschinen zu einer weiteren Prozessierung (Umhüllung, Konfektionierung usw.) oder Prüfung verarbeitbar; zum anderen wird ermöglicht, die Dickegradienten bereits durch die Form der Matrixschicht vorzugeben. Da diese zuerst gepresst wird, wozu sehr einfache Tablettenpressen verwendbar sind, kann bei dem wesentlich kritischeren, späteren Aufpressen der Abdeckschicht mit Hilfe von Spezialpressen konventionelles, relativ unproblematisches Presswerkzeug verwendet werden.

Eine bikonvexe Gesamtgeometrie vorausgesetzt, kann die erfindungsgemäße Schichttablette selbstverständlich auch in einem anschließenden Umhüllungsprozess mit einem Polymerfilm oder einer zuckerhaltigen Umhüllung versehen werden. Auf diese Weise lassen sich übelriechende oder -schmeckende Wirkstoffe maskieren, Tabletten kennzeichnen, Magensaftresistenz induzieren und weitere, beim Tablettencoating übliche Ziele verwirklichen.

Als Herstellungsverfahren für eine erfindungsgemäße Schichttablette ist nach der Erfindung die Kombination der folgenden Schritte vorgesehen: Zunächst wird eine wirkstoffhaltige Matrixschicht auf einer gängigen Exzenteroder Rundlaufpresse hergestellt, vorzugsweise mit einem speziellen Stempelwerkzeug, welches so ausgebildet ist, daß es bereits in den hierbei entstehenden Pressling Dickegradienten bewirkt, die komplementär zu den gewünschten Dickegradienten der späteren Abdeckschicht verlaufen. Für das Zusammenpressen mit dem pulver- oder granulatförmigen Material für die Abdeckschicht ist außer anderem Stempelwerkzeug auch die Verwendung von speziellen Tablettenmaschinen notwendig. Diese Spezialmaschinen müssen in der Lage sein, den Pressling aufzunehmen und ihn präzisse in eine Matrizenöffnung einzuführen, in welche sodann das Material für die Abdeckschicht gefüllt und beide Komponenten zusammengepresst werden können. Schichttablettenpressen sind hierzu nicht in der Lage, jedoch werden seit kurzem spezielle Manteltablettenpressen einer neuen Generation angeboten, welche vakuumbeaufschlagte Transferelemente zur Aufnahme von Presslingen und zu deren Transfer in Matrizenbohrungen besitzen. Diese Maschinen sind zur Fertigung der erfinungsgemäßen Schichttabletten aufgrund der Präzision, mit der sie die vorgefertigten Presslinge erfassen und bewegen können, besonders geeignet.

Prinzipiell kann die Herstellung auch in umgekehrter Reihenfolge geschehen, so daß zunächst die Abdeckschicht als Pressling gefertigt wird und dieser dann unter Zugabe von Pulver oder Granulat für die wirkstoffhaltige Matrixschicht zusammengepresst wird.

Eine Kombination der Schichttablette mit anderen Komponenten kann die erfindungsgemäße Schichttablette zu einer weiteren Arzneiform mit anderen Darreichungsformen ergänzen. Inbesondere ist die Kombination mit einem Therapie-System, welches eine verlängerte Wirkdauer im Magen besitzt, von Vorteil. So kann eine gesteuerte Wirkstofffreisetzung auch über einen längeren Zeitraum als bei bisherigen Retardformen erreicht werden, da die Aufenthaltsdauer im resorptionsaktiven Abschnitt des Gastrointestinaltraktes erhöht wird.

Eine vorteilhafte Ausgestaltung der Schichttablette kann auch dadurch gegeben sein, daß die Matrix aus mehr als einer Schicht besteht und jede dieser Schichten unterschiedliches Material aufweist und/oder unterschiedliche Wirkstoffe enthält.

Die Matrix kann ihrerseits im flüssigen Medium erodierbar oder nicht erodierbar sein, wobei im letzteren Falle der Wirkstoff durch Diffusion freigesetzt wird. Im Falle der Erosion der Matrix muss diese langsamer erfolgen als die Erosion der Abdeckschicht.
Auch kann eine wirkstoffhaltige Matrix Dickegradienten aufweisen, deren Richtung komplementär zur aufliegenden Abdeckschicht ist. Die erfindungsgemäße Schichttablette kann weiterhin zwei wirkstoffhaltige Matrices aufweisen, deren Kontaktflächen zum flüssigen Freisetzungsmedium unterschiedliche Abdeckschichten aufweise, wobei diese mit unterschiedlichen Dickegradienten ausgebildet sind und oder aus unterschiedlich erodierbaren Materialien bestehen können.

Schließlich Können sich die Dickegradienten über ihre Erstreckung kontinuierlich oder diskontinuierlich ändern.

Im folgenden wird die Erfindung anhand von Ausführungsbeispielen in der Zeichnung weiter erläutert. Es zeigen:
- Fig. 1a, 1b, 1c:: zum Vergleich typische Schichttabletten aus:
a) Hunnius Pharmazeutisches Wörterbuch
b) und c) Pharmaceutical Dosage Forms, Vol. 1 (Ed.H.A. Lieberman et al)
- Fig. 2 bis 4:: eine besonders günstige Ausbildung einer Schichttablette bzw. Teilen davon nach der Erfindung

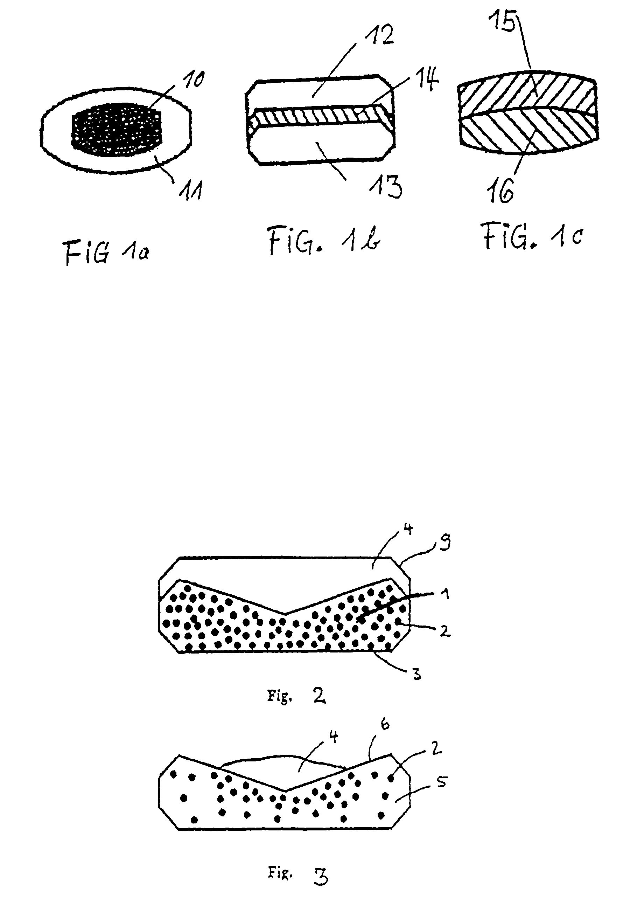

In der Fig. 1a ist eine sogenannte Manteltablette mit einem vorgepressten Kern (10) und einem ihn lückenlos umgebenden, ebenfalls durch Pressen von Pulver oder Granulat erzeugten Mantel (11) dargestellt. Kern (10) und/oder Mantel (11) können unterschiedliche Wirkstoffe enthalten. Wenn der Mantel (11) keinen Wirkstoff enthält, kann es sich um eine Retardform der Manteltablette handeln, wobei das Material des Mantels (11) beispielsweise in unterschiedlich sauren oder alkalischen Milieu des Magen-Darm-Traktes erodiert und danach Wirkstoff aus dem Kern (10) an unterschiedlichen Bereichen des Intestinaltraktes freigesetzt wird.
Ähnlich können die Schichten (12 bis 14) der Schichttablette 1b aufgebracht sein, und ebenfalls die Schichten (15,16) der Tablette nach Fig. 1c.

Fig. 2 zeigt ein Beispiel für eine besonders bevorzugte erfindungsgemäße Schichttablette. Es handelt sich hierbei um eine biplane Tablettenform mit Facettenrand (9). Die Schichttablette besitzt eine schichtförmige Matrix (1),welche Wirkstoff (2) enthält, und weist eine Oberfläche (3) auf, welche in einem flüssigen Medium die Kontaktfläche zwischen der wirkstoffhaltigen Matrix und dem Medium darstellt. Ein Teil der Ober- bzw. Kontaktfläche (3) weist eine auf die schichtförmige Matrix (1) aufgepresste, die Wirkstoffreisetzung verzögernde bzw. verhindernde Abdeckschicht (4) aus erodierbarem Material mit Dickegradienten auf.

In Fig. 3 wird der Zustand der erfindungsgemäßen Schichttablette aus Fig. 2 inmitten des Freisetzungsverlaufes gezeigt. Die von der Abdeckschicht (4) unbedeckten Randzonen (5) der schichtförmigen, wirkstoffhaltigen Matrix (1) haben einen Teil des Wirkstoffes (2) abgegeben; sie sind an Wirkstoff verarmt. Die Erosion der Abdeckschicht (4) hat an den Stellen geringer Dicke bereits zum Entstehen zusätzlicher Kontaktfläche (6) an der schichtförmigen Matrix geführt.

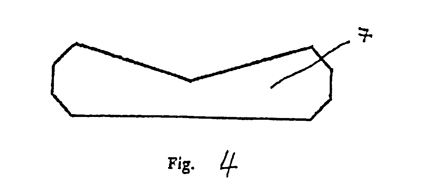

Fig. 4 zeigt beispielhaft einen möglichen Zustand nach abgeschlossener Wirkstofffreisetzung; Die nunmehr wirkstofffreie, schichtförmige Matrix (7) hat ihre die Freisetzung verzögernde Abdeckung durch Erosion vollständig verloren.

## Patentansprüche

1. Verfahren zur Herstellung einer Schichttablette zur kontrollierten Freisetzung von Wirkstoffen in einem flüssigen Medium mit mindestens einer wirkstoffhaltigen, Kontaktflächen gegenüber dem flüssigen Medium aufweisenden schichtförmigen Matrix, deren Kontaktflächen zum Teil eine die Wirkstofffreisetzung verzögernde oder verhindernde Abdeckschicht aufweisen, wobei die wirkstoffhaltige Matrix und die erodierbare Abdeckschicht Dickegradienten besitzen, deren Richtungen komplementär zueinander verlaufen, **dadurch gekennzeichnet, daß**
a) mittels eines speziell geformten Stempelwerkzeugs die Matrix oder die erodierbare Abdeckschicht mit einem Dickegradienten als erster Teilkörper erzeugt wird, der
b) mit vakuumbeaufschlagten Transferelementen in die Matrizenöffnung einer Tablettiermaschine transferiert wird, und
c) das Pulver- oder Granulat-Material für die den ersten Teilkörper ergänzende(n) Abdeckschicht(en) oder Matrix zum ersten Teilkörper gegeben und zusammengepresst wird.

2. Verfahren nach Anspruch 1, **dadurch gekennzeichnet, daß** die Abdeckschicht als Teilkörper aus mehr als einer Schicht gebildet wird.

3. Verfahren nach Anspruch 1 oder 2, **dadurch gekennzeichnet, daß** die wirkstoffhaltige Matrix als Teilkörper aus mehr als einer Schicht gebildet wird, wobei jede dieser Schichten unterschiedliches Material aufweist und/oder unterschiedliche Wirkstoffe enthält.

4. Verfahren nach Anspruch 3, welches zwei wirkstoffhaltige Matrices aufweist, **dadurch gekennzeichnet, daß** auf den unterschiedlichen Kontaktflächen zum flüssigen Freisetzungsmedium einer zweischichtigen Matrix unterschiedliche Abdeckschichten erzeugt werden, die unterschiedliche Dickegradienten aufweisen und/oder aus unterschiedlich erodierbarem Material bestehen.

5. Verfahren nach einem der Ansprüche 1 bis 4, **dadurch gekennzeichnet, daß** sich die Dickegradienten über ihre Erstreckung kontinuierlich oder diskontinuierlich ändern.

6. Verfahren nach einem der vorangehenden Ansprüche, **dadurch gekennzeichnet, daß** die Schichttablette als Bestandteil einer Arzneiform mit verlängerter Verweildauer im Magen konzipiert wird.

7. Verfahren nach einem der vorangehenden Ansprüche, **dadurch gekennzeichnet, daß** die Schichttablette mit einem Polymerfilm überzogen wird.

8. Verfahren nach einem der vorangehenden Ansprüche, **dadurch gekennzeichnet, daß** die Schichttablette mit einem pharmazeutischen Wirkstoff in der Matrix gebildet wird.

9. Verwendung des Verfahrens nach einem der Ansprüche 1 bis 8 zur Herstellung einer Schichttablette zur kontrollierten Freisetzung von Wirkstoffen in einem flüssigen Medium, mit mindestens einer wirkstoffhaltigen, Kontaktflächen gegenüber dem flüssigen Medium aufweisenden schichtförmigen Matrix, deren Kontaktflächen zum Teil eine die Wirkstofffreisetzung verzögernde oder verhindernde Abdeckschicht aufweisen, wobei besagte Schichttablette eine biplanare Tablettenform mit Facettenrand aufweist, deren an die Abdeckschicht angrenzende Matrixgrenzfläche anschließend an einen Verlauf parallel zu einer von der Abdeckschicht gebildeten Facettenfläche eine konisch zum Tablettenzentrum hin abfallende Senke aufweist (Fig. 2).

## Claims

1. Process for the production of a layered tablet for the controlled release of active substances in a liquid medium, comprising at least one active substance-containing, layered matrix which has contact surfaces to the liquid medium, said contact surfaces being partially provided with a cover layer delaying or preventing the active substance release, the said active substance-containing matrix and the said erodible cover layer possessing thickness gradients the directions of which are complementary to each other, **characterized in that**
a) the matrix or the erodible cover layer is formed, with a thickness gradient, as the first component body, by means of a specially formed punch, which component body is
b) transferred into the die opening of a tabletting machine by using transfer elements to which a vacuum has been applied, and
c) the powder or granulate material for the cover layer(s) or matrix which complement(s) the first partial body is added to the first partial body and compressed.

2. Process according to Claim 1, **characterized in that** the cover layer is formed as a component body having more than one layer.

3. Process according to Claim 1 or 2, **characterized in that** the active substance-containing matrix, as a component body, is formed having more than one layer, each of these layers being of a different material and/or containing different active substances.

4. Process according to Claim 3 involving two active substance-containing matrices, **characterized in that** on the different contact surfaces of a double-layered matrix to the liquid release medium there are formed different cover layers which have different thickness gradients and/or consist of different erodible material.

5. Process according to any one of Claims 1 to 4, **characterized in that** the thickness gradients vary continuously or discontinuously over their extent.

6. Process according to one of the preceding Claims, **characterized in that** the layered tablet is designed as a component of a drug having prolonged retention time in the stomach.

7. Process according to one of the preceding Claims, **characterized in that** the layered tablet is coated with a polymer film.

8. Process according to one of the preceding Claims, **characterized in that** the layered tablet is formed with a pharmaceutical active substance in the matrix.

9. Use of the process according to any one of Claims 1 to 8 for making a layered tablet for controlled release of active agents in a liquid medium which has at least one active substance-containing, layered matrix having contact surfaces to the liquid medium, which contact surfaces are partially provided with a cover layer delaying or preventing the release of active substance, the said layered tablet having a biplanar tablet form with a facet margin whose matrix interface adjacent to the cover layer has a depression which follows after a portion running parallel to a facet surface formed by the cover layer, and which depression tapers toward the centre of the tablet.

## Revendications

1. Procédé de fabrication d'un comprimé stratifié pour la libération contrôlée de substances actives dans un milieu liquide avec au moins une matrice stratiforme, contenant des substances actives, présentant des surfaces de contact par rapport au milieu liquide, dont les surfaces de contact présentent en partie une couche de recouvrement retardant ou empêchant la libération des substances actives, la matrice contenant des substances actives et la couche de recouvrement érodable possédant des gradients d'épaisseur, dont les sens passent de façon complémentaire les uns par rapport aux autres, **caractérisé en ce que**
a) à l'aide d'un outil de poinçonnage de forme spéciale, la matrice ou la couche de recouvrement érodable est créée avec un gradient d'épaisseur comme premier corps partiel qui
b) est transféré avec des éléments de transfert, auxquels du vide est appliqué, dans l'ouverture de la matrice d'une machine de production de comprimés, et
c) la matière en poudre ou en granulés pour la/les couche(s) de recouvrement ou matrice complétant le premier corps partiel est donnée pour le premier corps partiel et est comprimée.

2. Procédé selon la revendication 1, **caractérisé en ce que** la couche de recouvrement est formée comme corps partiel composé de plus d'une couche.

3. Procédé selon la revendication 1 ou 2, **caractérisé en ce que** la matrice contenant des substances actives comme corps partiel est formée de plus d'une couche, chacune de ces couches présentant une matière différente et/ou contenant diverses substances actives.

4. Procédé selon la revendication 3, lequel présente deux matrices contenant des substances actives, **caractérisé en ce que** sur les diverses surfaces de contact pour un milieu de libération liquide d'une matrice à deux couches, des couches de recouvrement différentes sont produites, lesquelles présentent différents gradients d'épaisseur et/ou se composent d'une matière diversement érodable.

5. Procédé selon l'une des revendications 1 à 4, **caractérisé en ce que** les gradients d'épaisseur se modifient sur leur extension de façon continue ou discontinue.

6. Procédé selon l'une des revendications précédentes, **caractérisé en ce que** le comprimé stratifié est conçu comme partie constitutive d'une forme médicamenteuse avec une durée de séjour prolongée dans l'estomac.

7. Procédé selon l'une des revendications précédentes, **caractérisé en ce que** le comprimé stratifié est recouvert d'une pellicule polymère.

8. Procédé selon l'une des revendications précédentes, **caractérisé en ce que** le comprimé stratifié est formé d'une substance active pharmaceutique dans la matrice.

9. Utilisation du procédé selon l'une des revendications 1 à 8 pour la fabrication d'un comprimé stratifié pour la libération contrôlée de substances actives dans un milieu liquide, avec au moins une matrice stratiforme, contenant des substances actives, présentant des surfaces de contact par rapport au milieu liquide, lesdites surfaces de contact présentant en partie une couche de recouvrement retardant ou empêchant la libération des substances actives, le comprimé stratifié susmentionné présentant une forme de comprimé biplane avec un bord en biseau, dont la surface limite de matrice adjacente à la couche de recouvrement présente un abaissement incliné de façon conique vers le centre du comprimé faisant suite à un tracé parallèle à une surface en biseau formée par la couche de recouvrement (figure 2).
